# EUROPEAN PATENT APPLICATION

(11) **EP 4 534 149 A1**
(43) Date of publication of application: **09.04.2025**
(21) Application number: 23811886.3
(22) Date of filing: 25.05.2023
(51) Int. Cl.: A61Q 1/00, A61Q 15/00, A61K 8/02, A61K 8/34, A61K 8/36, A61K 8/365, A61K 8/44

(54) **GELATION AGENT COMPOSITION**

(30) Priority: 26.05.2022 JP 2022086341
(71) Applicant: AJINOMOTO CO., INC., Chuo-ku Tokyo 104-8315 (JP)
(72) Inventor: IKEDA, Shiori, Kawasaki-shi, Kanagawa 210-8681 (JP)
(74) Representative: Mewburn Ellis LLP
(86) International application number: PCT/JP2023/019544
(87) International publication number: WO 2023/229014

(57) **Abstract**

The present invention relates to a gelling agent composition containing (A) to (C) with respect to the total amount of the composition:
(A) dibutyl lauroyl glutamide: 5 to 40% by weight, (B) an acyl acidic amino acid with an acyl group having 1 to 22 carbon atoms, or hydroxy acid: 0.4 to 22% by weight, and (C) a polar oil: 37 to 90% by weight, wherein a weight ratio (B)/(A) is 0.03 to 0.7. The gelling agent composition of the present invention has an appropriate melting temperature, does not require excessive heating, and is most suitable for the production of cosmetic products, and the like. Furthermore, by preparing using the present gelling agent composition, a gelatinous composition free of rising of oil over time can be provided, and even when formed into a stick shape, sufficient gel strength (breaking strength) and superior texture can be imparted. Thus, the composition is most suitable for cosmetic products and the like.

## Description

### [Technical Field]

The present invention relates to gelling agent compositions and gelatinous compositions containing same, and methods for suppressing sweating of gelatinous compositions.

### [Background Art]

Amino acid-based gelling agents such as dibutyl N-lauroyl-L-glutamide and dibutyl N-2-ethylhexanoyl glutamide are capable of gelling oils and are used as stabilizers for solid cosmetics, emulsion compositions and the like. On the other hand, it is known that, when gelling an oil by using an amino acid-based gelling agent, heating at very high temperature is necessary to uniformly dissolve these gelling agents in oil and the handling in manufacturing is difficult. In order to solve this problem, attempts have been made to dissolve the gelling agent beforehand in higher alcohols or fatty acids, which are superior in dissolving them, and then use the oil gelling agent composition to gel oil-containing formulations. However, gelling agent compositions using these solvents have problems that they cannot maintain sufficient gel strength when combined with other liquid oils to prepare oil formulations, and that oil floats out over time.

For example, a gelatinous composition with an extremely low melting temperature has been provided, in which polyhydric alcohol and polar oil are blended with an N-acyl acidic amino acid dialkylamide. However, because the emphasis was placed on the melting temperature, there remained issues regarding oil separation over time and gel strength when blended into a solid formulation (Patent Literature 1).

In addition, by using a fatty acid having 3 to 22 carbon atoms as a solvent, a gelatinous composition has been provided that can be melted at a temperature considerably lower than that of a general solvent, and can prepare an oily formulation with good stability (no sweating phenomenon). However, because the emphasis was placed on the melting temperature, there has been left a problem that the gel strength of the solid formulation is insufficient (Patent Literature 2).

Furthermore, by combining a transparent gelling agent with an oil agent having a high refractive index and an oil agent having a low volume expansion rate, a transparent oilbased solid cosmetic with superior transparency, no perspiration property, high storage stability, and a pleasant feel when used has been provided. However, there are not many oil agents with a low volume expansion rate, which are essential components, and it has been practically difficult to assemble a formulation (Patent Literature 3).

### [Citation List]

### [Patent Literature]

[Patent Literature 1]
   Japanese Patent No. 6834178
[Patent Literature 2]
   JP-2018-154809 A
[Patent Literature 3]
   Japanese Patent No. 6503008

### [Summary of Invention]

### [Technical Problem]

The problem of the present invention is to provide a gelling agent having an appropriate melting temperature which does not cause rising of oil (sweating phenomenon) over time, can maintain sufficient breaking strength even when molded into a stick shape, and can prepare a gelatinous composition such as a solid cosmetic having a superior texture.

### [Solution to Problem]

In view of the above-mentioned problems, the present inventors have conducted intensive studies and found that a composition containing specific amounts of (A) dibutyl lauroyl glutamide, (B) acyl acidic amino acid with an acyl group having 1 to 22 carbon atoms, or hydroxy acid, and (C) polar oil at a specific ratio has a low melting temperature, and solid cosmetics and the like prepared using the composition show suppressed sweating phenomenon and have optimal gel strength and texture for the cosmetics, which resulted in the completion of the present invention.

That is, the present invention includes the following embodiments.
[1] A gelling agent composition comprising the following (A) to (C) with respect to the total amount of the composition
   (A) dibutyl lauroyl glutamide: 5 to 40% by weight,
   (B) an acyl acidic amino acid with an acyl group having 1 to 22 carbon atoms, or hydroxy acid: 0.4 to 22% by weight, and
   (C) a polar oil: 37 to 90% by weight,
   wherein a weight ratio (B)/(A) is 0.03 to 0.7.
[2] The gelling agent composition of [1], further comprising (D) a polyhydric alcohol and comprising, with respect to the total amount of the composition,
   (A) dibutyl lauroyl glutamide: 20 to 40% by weight,
   (B) an acyl acidic amino acid with an acyl group having 1 to 22 carbon atoms, or hydroxy acid: 1 to 22% by weight,
   (C) a polar oil: 37 to 70% by weight, and
   (D) a polyhydric alcohol: 5 to 20% by weight
   wherein a weight ratio (D)/(A) is less than 0.5.
[2-1] The gelling agent composition of [1] or [2], wherein the (B) is an acyl acidic amino acid with an acyl group having 1 to 22 carbon atoms.
[3] The gelling agent composition of any of [1] to [2-1], wherein the (B) acidic amino acid is glutamic acid.
[4] The gelling agent composition of any of [1] to [3], wherein the (B) acyl acidic amino acid is at least one type selected from the group consisting of N-lauroyl glutamic acid, N-myristoyl glutamic acid, N-palmitoyl glutamic acid, N-stearoyl glutamic acid, and N-cocoyl glutamic acid.
[5] The gelling agent composition of [1] or [2], wherein the (B) hydroxy acid is at least one type selected from the group consisting of lactic acid, citric acid, and malic acid.
[6] The gelling agent composition of any of [1] to [5], wherein the (C) polar oil is at least one type selected from a fatty acid having 8 to 22 carbon atoms and a higher alcohol having 8 to 24 carbon atoms.
[7] The gelling agent composition of [6], wherein the (C) polar oil is at least one type selected from isostearyl alcohol, 2-hexyldecanol, 2-octyldodecanol, oleyl alcohol, 2-decyltetradecanol, isostearic acid, and stearic acid.
[8] The gelling agent composition of any of [2] to [7], wherein the weight ratio (D)/(A) is 0.1 to 0.45.
[9] The gelling agent composition of any of [2] to [8], wherein the (D) polyhydric alcohol is a polyhydric alcohol having 2 to 12 carbon atoms.
[10] The gelling agent composition of any of [2] to [9], wherein the (D) polyhydric alcohol is at least one type selected from the group consisting of butylene glycol, propylene glycol, propanediol, pentylene glycol, isopentyldiol, hexanediol, and dipropylene glycol.
[11] The gelling agent composition of any of [1] to [10], which is used for a solid cosmetic.
[12] The gelling agent composition of [11], wherein the solid cosmetic is a stick-shaped cosmetic.
[13] The gelling agent composition of any of [1] to [12], which melts at 99°C or lower.
[14] A gelatinous composition comprising the gelling agent composition of any of [1] to [13] and an oil agent.
[15] The gelatinous composition of [14], comprising 5 to 50% by weight of the gelling agent composition relative to the total weight of the gelatinous composition.
[16] The gelatinous composition of [14] or [15], having a rupture strength of 1500 g/cm² or more and 6600 g/cm² or less as measured at an insertion speed of 6 cm/min.
[17] The gelatinous composition of any of [14] to [16], which is a stick-shaped cosmetic.
[18] The gelling agent composition of [1], comprising, with respect to the total amount of the composition,
   (A) dibutyl lauroyl glutamide: 5 to 20% by weight,
   (B) an acyl acidic amino acid with an acyl group having 1 to 22 carbon atoms: 0.4 to 12% by weight, and
   (C) a polar oil: 65 to 90% by weight
   wherein a weight ratio (B)/(A) is 0.03 to 0.7.
[19] The gelling agent composition of [18], wherein the (B) acidic amino acid is glutamic acid.
[20] The gelling agent composition of [18], wherein the (B) acyl acidic amino acid is at least one type selected from the group consisting of N-lauroyl glutamic acid, N-myristoyl glutamic acid, N-palmitoyl glutamic acid, N-stearoyl glutamic acid, and N-cocoyl glutamic acid.
[21] The gelling agent composition of any of [18] to [20], wherein the (C) polar oil is at least one type selected from a fatty acid having 8 to 22 carbon atoms and a higher alcohol having 8 to 24 carbon atoms.
[22] The gelling agent composition of [21], wherein the (C) polar oil is at least one selected from isostearyl alcohol, 2-hexyldecanol, 2-octyldodecanol, oleyl alcohol, 2-decyltetradecanol, isostearic acid, and stearic acid.
[23] The gelling agent composition of any of [18] to [22], further comprising (D) a polyhydric alcohol.
[24] The gelling agent of [23], wherein a weight ratio (D)/(A) is less than 0.5.
[25] The gelling agent of [23], wherein the (D) polyhydric alcohol is contained at 0.01 to 10% by weight with respect to the total amount of the composition and a weight ratio (D)/(A) is 0.1 to 0.45.
[26] The gelling agent composition of any of [23] to [25], wherein the (D) polyhydric alcohol is a polyhydric alcohol having 2 to 12 carbon atoms.
[27] The gelling agent composition of any of [23] to [26], wherein the (D) polyhydric alcohol is at least one type selected from the group consisting of butylene glycol, propylene glycol, propanediol, pentylene glycol, isopentyldiol, hexanediol, and dipropylene glycol.
[28] The gelling agent composition of any of [18] to [27], which is used for a solid cosmetic.
[29] The gelling agent composition of [28], wherein the solid cosmetic is a stick-shaped cosmetic.
[30] The gelling agent composition of any of [18] to [29], which melts at 100°C or lower.
[31] A gelatinous composition comprising the gelling agent composition of any of [18] to [30] and an oil agent.
[32] The gelatinous composition of [31], comprising 40 to 75% by weight of the gelling agent composition relative to the total weight of the gelatinous composition.
[33] The gelatinous composition of [31] or [32], having a rupture strength of 1500 g/cm² or more and 6500 g/cm² or less as measured at an insertion speed of 6 cm/min.
[34] The gelatinous composition of any of [31] to [33], which is a stick-shaped cosmetic.
[35] A method for suppressing sweating in a solid cosmetic, comprising blending the gelling agent composition of [1] in an oil agent.
[36] A method for suppressing sweating in a solid cosmetic, comprising blending the gelling agent composition of [2] or [18] in an oil agent.
[37] A thickener composition comprising the following (A) to (D) with respect to the total amount of the composition
   (A) dibutyl lauroyl glutamide: 20 to 40% by weight,
   (B) an acyl acidic amino acid with an acyl group having 1 to 22 carbon atoms: 1 to 22% by weight,
   (C) a polar oil: 37 to 70% by weight, and
   (D) a polyhydric alcohol: 5 to 20% by weight
   wherein a weight ratio (D)/(A) is less than 0.5.
[38] The thickener composition of [37], wherein the (B) acyl acidic amino acid is at least one type selected from the group consisting of N-lauroyl glutamic acid, N-myristoyl glutamic acid, N-palmitoyl glutamic acid, N-stearoyl glutamic acid, and N-cocoyl glutamic acid.
[39] The gelling agent composition of [37] or [38], wherein the (C) polar oil is at least one type selected from isostearyl alcohol, 2-hexyldecanol, 2-octyldodecanol, oleyl alcohol, 2-decyltetradecanol, isostearic acid, and stearic acid.
[40] The thickener composition of any of [37] to [39], wherein a weight ratio (D)/(A) is 0.1 to 0.45.
[41] The thickener composition of any of [37] to [40], wherein the (D) polyhydric alcohol is at least one type selected from the group consisting of butylene glycol, propylene glycol, propanediol, pentylene glycol, isopentyldiol, hexanediol, and dipropylene glycol.
[42] The thickener composition of any of [37] to [41], which is used for an emulsion cosmetic.
[43] The thickener composition of [42], wherein the emulsion cosmetic is milky lotion.
[44] The gelling agent composition of any of [37] to [43], which melts at 99°C or lower.
[45] An emulsion composition comprising the thickener composition of any of [37] to [44] and an oil agent.
[46] The emulsion composition of [45], which is milky lotion.

### [Advantageous Effects of Invention]

According to the present invention, a cosmetic that can be prepared into a stick-shaped cosmetic capable of maintaining a sufficient breaking strength, and is superior in moldability and texture can be provided.

According to the present invention, a solid cosmetic that can be combined with various oil agents, suppresses rising of oil over time, and is superior in stability can be provided.

When the gelling agent composition of the present invention is used for producing cosmetic products and perfumery, a conventional treatment at a very high temperature is not necessary, easy production is possible, and production steps of cosmetic products and the like can be simplified drastically.

### [Description of Embodiments]

The present invention relates to a gelling agent composition containing the following (A) to (C), and respective contents with respect to the total weight of the composition are
(A) dibutyl lauroyl glutamide: 5 to 40% by weight,
(B) acyl acidic amino acid with an acyl group having 1 to 22 carbon atoms, or hydroxy acid: 0.4 to 22% by weight, and
(C) polar oil: 37 to 90% by weight,
wherein a weight ratio (B)/(A) is 0.03 to 0.7 (also to be referred to as the gelling agent composition of the present invention).

### (A) dibutyl lauroyl glutamide

Dibutyl lauroyl glutamide in the present invention is represented by the following formula, and is also called N-lauroyl glutamic acid dibutyl amide.

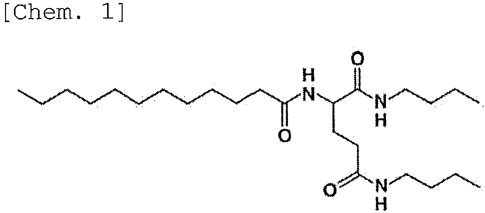

Dibutyl lauroyl glutamide can be produced, for example, by reacting a long-chain fatty acid halide with L-glutamic acid in the presence of a basic catalyst by the Schotten-Baumann reaction to produce N-acylated glutamic acid, followed by heating and reacting an amine derivative such as alkylamine and the like in the presence or absence of an acid catalyst. Alternatively, for example, it can be produced by reacting glutamic acid with an amine derivative such as alkylamine and the like in the presence or absence of an acid catalyst, and then N-acylating the obtained glutamic acid diamide with an acylating agent such as fatty acid halide and the like. Dibutyl lauroyl glutamide can be produced by the aforementioned method, or can be commercially available from Ajinomoto Co., Inc. (trade name "GP-1").

### (B) acyl acidic amino acid with acyl group having 1 to 22 carbon atoms, or hydroxy acid

In the present invention, an acyl acidic amino acid with an acyl group having 1 to 22 carbon atoms may be a free form or a salt thereof, and a free form is preferred. The acyl acidic amino acid may be any of D-form, L-form and DL-form. These N-acyl acidic amino acids may be used alone or in a combination of two or more kinds thereof in any ratio.

The acyl group of the acyl acidic amino acid with an acyl group having 1 to 22 carbon atoms in the present invention is an acyl group derived from a fatty acid having 1 to 22 carbon atoms, preferably an acyl group derived from a fatty acid having 8 to 22 carbon atoms, more preferably an acyl group derived from a fatty acid having 8 to 18 carbon atoms, further preferably an acyl group derived from a fatty acid having 12 to 18 carbon atoms. The acyl group may be a saturated acyl group or an unsaturated acyl group, and a saturated acyl group is preferred. Examples of the acyl group include acyl groups derived from lauric acid, myristic acid, palmitic acid, stearic acid, oleic acid, isostearic acid and the like, and mixtures thereof such as beef tallow fatty acid, coconut oil fatty acid, palm kernel oil fatty acid, and the like, and an acyl group derived from lauric acid, myristic acid, palmitic acid, stearic acid, or coconut oil fatty acid is preferred.

The acidic amino acid in (B) in the present invention is not particularly limited as long as it is an acidic amino acid. From the aspects of sweating suppression, lower melting temperature, and texture, examples thereof include glutamic acid, aspartic acid, and the like, and glutamic acid is preferred.

Examples of the salt of acyl acidic amino acid with an acyl group having 1 to 22 carbon atoms include alkali metal salts such as lithium salts, sodium salts, and potassium salts; alkaline earth metal salts such as calcium salts and magnesium salts; alkanolamine salts such as monoethanolamine salts, diethanolamine salts, and triethanolamine (TEA) salts; ammonium salts; and basic organic salts.

Specific examples of the N-acyl acidic amino acid with an acyl group having 1 to 22 carbon atoms or a salt thereof to be used in the present invention include N-lauroyl glutamic acid, N-myristoyl glutamic acid, N-palmitoyl glutamic acid, N-stearoyl glutamic acid, N-cocoyl (coconut oil fatty acid acyl) glutamic acid, N-lauroyl aspartic acid, N-myristoyl aspartic acid, N-palmitoyl aspartic acid, N-stearoyl aspartic acid, N-cocoyl aspartic acid, and mono- or di-sodium salts, mono- or di-potassium salts, mono- or di-triethanolamine salts, and the like thereof. These may be used alone or in combination of two or more kinds thereof. Among these, from the aspects of sweating suppression and texture, N-lauroyl glutamic acid, N-myristoyl glutamic acid, N-palmitoyl glutamic acid, N-stearoyl glutamic acid, and N-cocoyl (coconut oil fatty acid acyl) glutamic acid are preferred.

In the present invention, the hydroxy acid may be a free form or a salt thereof, and examples of the hydroxy acid include α-hydroxy acids such as gluconic acid, citric acid, isocitrate, alloisocitric acid, lactic acid, phenyllactic acid, hydroxyacetic acid, malic acid, and tartaric acid, and β-hydroxy acids such as salicylic acid, substituted salicylic acid, 3-hydroxybutyric acid, 3-hydroxyvaleric acid, and 3-hydroxycaproic acid. These may be used alone or in a combination of two or more kinds thereof. Among these, from the aspects of sweating suppression and texture, lactic acid, citric acid, malic acid, tartaric acid, salicylic acid, hydroxyacetic acid, and the like are preferred, and lactic acid, citric acid, and malic acid are more preferred.

### (C) polar oil

The polar oil in the present invention is not particularly limited as long as it is an oil having an inorganic value of 20 or more in the organic conceptual diagram. It is preferably at least one selected from fatty acids having 8 to 22 carbon atoms and higher alcohols having 8 to 24 carbon atoms.

The number of carbon atoms of the fatty acid is generally 8 to 22, preferably 8 to 20, more preferably 8 to 18, from the aspects of the stability of formulation, compatibility with an oil agent and lowering of the melting temperature of the composition. Specific examples of the saturated fatty acid include straight chain saturated fatty acids such as octanoic acid (caprylic acid), nonanoic acid, decanoic acid (capric acid), dodecanoic acid (lauric acid), tetradecanoic acid (myristic acid), pentadecanoic acid, hexadecanoic acid (palmitic acid), heptadecanoic acid, octadecanoic acid (stearic acid), icosanoic acid (arachidic acid), henicosyl acid, docosanoic acid (behenic acid); and branched chain saturated fatty acids such as isostearic acid, 2-hexyldecanoic acid, 2-ethylhexanoic acid, isononanoic acid, 12-hydroxystearic acid. Among these, palmitic acid, stearic acid, isostearic acid and the like are preferred.

Examples of the unsaturated fatty acid include 9-hexadecenoic acid (pulmitoleic acid), cis-9-octadecenoic acid (oleic acid), 11-octadecenoic acid (vaccenic acid), cis,cis-9,12-octadecadienoic acid (linoleic acid), 9,12,15-octadecatrienoic acid ((9,12,15)-linolenic acid), 5,8,11,14-eicosatetraenoic acid (arachidonic acid). Among these, oleic acid is preferred.

Among the aforementioned fatty acids, hexadecanoic acid (palmitic acid), stearic acid, isostearic acid, and oleic acid are more preferred.

The number of carbon atoms of the higher alcohol is generally 8 to 24, preferably 12 to 24, more preferably 16 to 24, further preferably 16 to 20, from the aspects of formulation stability, compatibility with an oil agent, and lowering of the melting temperature of the composition. Specifically, lauryl alcohol, myristyl alcohol, 2-hexyldecanol, 2-octyldodecanol, oleyl alcohol, isostearyl alcohol, 2-decyltetradecanol, cetyl alcohol and the like can be mentioned, and isostearyl alcohol, 2-hexyldecanol, 2-octyldodecanol, oleyl alcohol, 2-decyltetradecanol, cetyl alcohol and the like are preferred.

From the aspect of melting temperature, (C) in the present invention is preferably at least one selected from isostearyl alcohol, 2-hexyldecanol, 2-octyldodecanol, oleyl alcohol, 2-decyltetradecanol, isostearic acid, and stearic acid.

The content of each component in the gelling agent composition of the present invention relative to the total weight of the composition and the ratio of each component are as follows.

From the aspects of gel strength and melting temperature, the content of (A) is generally 5% by weight or more, preferably 10% by weight or more, and the upper limit is 40% by weight or less, preferably 35% by weight or less, relative to the total weight of the composition.

From the aspect of suppression of sweating, the content of (B) is generally 0.4% by weight or more, preferably 0.5% by weight or more, and the upper limit is 22% by weight or less, preferably 21% by weight or less, relative to the total weight of the composition.

From the aspect of melting temperature, the content of (C) is generally 37% by weight or more, preferably 38% by weight or more, and the upper limit is 90% by weight or less, preferably 85% by weight or less, relative to the total weight of the composition.

From the aspect of suppression of sweating, the weight ratio (B)/(A) is generally 0.03 or more, preferably 0.035 or more, and the upper limit is 0.7 or less, preferably 0.68 or less.

While the form of the gelling agent composition of the present invention is not particularly limited, it can be provided as, for example, gel, particles, solid (rod-shape, sphere, sheet) or the like. Among these, gel or sphere is preferred.

The method for producing the gelling agent composition of the present invention is not particularly limited. For example, the target gelling agent composition can be obtained by heating and stirring a mixture containing (A) to (C) until a homogeneous solution is formed, and then cooling the mixture. The heating temperature varies depending on the types and contents of (A) to (C). The gelling agent composition can be obtained, for example, by mixing and heating the mixture at 85 to 130°C using an oil bath or heater for 5 to 30 minutes, and then leaving same at room temperature.

The gelling agent composition of the present invention may further contain (D) polyhydric alcohol.

### (D) polyhydric alcohol

The polyhydric alcohol in the present invention is not particularly limited as long as it has two or more hydroxyl groups, and preferably has two hydroxyl groups. The number of carbon atoms in the polyhydric alcohol is not particularly limited. It is preferably 2 to 12 carbon atoms, more preferably 2 to 6 carbon atoms, further preferably 3 to 6 carbon atoms, particularly preferably 3 to 5 carbon atoms. Examples thereof include ethylene glycol, dipropylene glycol, butylene glycol, glyceryl monocaprylate, caprylyl glycol, glycerin, diglycerin, propylene glycol, 1,3-propanediol, pentylene glycol, 1,5-pentanediol, 1,6-hexanediol, 1,2-hexanediol, hexylene glycol, 1,3-butylene glycol, neopentyl glycol, low-polymerized polyethylene glycol, ethylhexylglycerin, and isopentyldiol. From the aspect of lowering the melting temperature and sweating suppression, propylene glycol, pentylene glycol, propanediol, 1,3-butylene glycol, isopentyldiol, hexanediol, and dipropylene glycol are preferred, and propylene glycol, pentylene glycol, and 1,3-butylene glycol are more preferred.

From the aspects of lower melting temperature and sweating suppression, the content of (D) is generally 0.01% by weight or more, preferably 0.03% by weight or more, and the upper limit is 20% by weight or less, preferably 15% by weight or less, more preferably 10% by weight or less, relative to the total weight of the composition.

Since the gelling agent composition of the present invention can impart optimal gel strength to solid cosmetics as described below, it can be used as a gelling agent composition for solid cosmetics, preferably for stick-shaped cosmetics.

A gelatinous composition obtained by adding the gelling agent composition of the present invention to (E) an oil agent is also included in the present invention (also to be referred to as the gelatinous composition of the present invention).

### (E) oil agent

The oil agent in the present invention can be any oil agent that is generally used in cosmetic products, pharmaceutical products, and the like, without any particular restrictions. Examples include liquid oil agents, semi-solid oil agents, solid oil agents, and the like, and liquid oil agents and semi-solid oil agents are preferred.

Specific examples of the liquid oil agent include linear or branched hydrocarbon oils such as liquid paraffin, light isoparaffin, liquid isoparaffin, hydrogenated polyisobutene, squalane, squalene, and α-olefin oligomers; vegetable oils such as almond oil, jojoba oil, olive oil, jojoba seed oil, corn germ oil, wheat germ oil, meadowfoam oil, sunflower oil, and macadamia nut oil; animal fats and oils such as liquid lanolin; diisostearyl malate, isopropyl myristate, ester oils such as fatty acid esters and polyhydric alcohol fatty acid esters, including diisostearyl malate, isopropyl myristate, isotridecyl isononanoate, cetyl ethylhexanoate, ethylhexyl palmitate, isopropyl palmitate, tri(caprylic acid/capric acid)glyceryl, triisostearin, and triethylhexanoin; acyl amino acid esters such as isopropyl lauroyl sarcosine (Eldew (registered trademark) SL-205), di(cholesteryl/octyldodecyl) N-lauroyl-L-glutamate, hexyldecyl myristoyl methylaminopropionate, dihexyldecyl lauroyl glutamate, diisostearyl lauroyl glutamate, dioctyldodecyl lauroyl glutamate, bis(hexyldecyl/octyldodecyl) lauroyl glutamate, dioctyldodecyl lauroyl glutamate, and dioctyldodecyl stearoyl glutamate; Phytosterol esters such as di(phytosteryl/2-octyldodecyl) N-lauroyl-L-glutamate; silicone oils such as cyclohexasiloxane, cyclopentasiloxane, dimethylpolysiloxane, dimethylcyclopolysiloxane, methylphenylpolysiloxane, methylhydrogenpolysiloxane, higher alcohol-modified organopolysiloxane, and bisphenylpropyldimethicone, and fluorine oils such as fluoropolyethers and perfluoroalkyl ether silicones; and the like.

Specific examples of the semi-solid oil agent include cholesterol esters such as cholesteryl isostearate, cholesteryl hydroxystearate, cholesteryl macadamia nut oil fatty acid, di(cholesteryl/behenyl/octyldodecyl) N-lauroyl-L-glutamate; phytosterol esters such as di(phytosteryl/behenyl/2-octyldodecyl) N-lauroyl-L-glutamate, (phytosteryl/decyltetradecyl) myristoyl methyl-β-alanine isostearate, phytosteryl oleate; dipentaerythritol fatty acid esters such as dipentaerythritol hexaoxystearate, dipentaerythritol rosinate; triglycerides such as caprylic/capric/myristic/stearic triglyceride; partially hydrogenated triglycerides such as hydrogenated oils; lanolin, lanosterols, petrolatum, and the like.

Specific examples of the solid oil agent include animal waxes, vegetable waxes, mineral waxes, synthetic waxes, and the like, specifically, rice bran wax, carnauba wax, candelilla wax, beeswax, porphyrin wax, ceresin, shea butter, cetyl palmitate, solid paraffin, microcrystalline wax, polyethylene wax, polyolefin wax, and the like.

The above-mentioned oil agents may be used alone or in a combination of two or more kinds thereof. Among these, from the aspects of broad utility and texture, hydrocarbon oils such as liquid paraffin, hydrogenated polyisobutene, α-olefin oligomer, fatty acid ester oils such as diisostearyl malate, isopropyl myristate, isotridecyl isononanoate, triethylhexanoin, and phytosterol esters such as N-lauroyl-L-glutamic acid di(phytosteryl/2-octyldodecyl) and N-lauroyl-L-glutamic acid di(phytosteryl/behenyl/2-octyldodecyl) are preferred. In particular, it is known that phytosterol esters have the effect of markedly improving gel strength, and it is particularly desirable to contain 2 to 10% of liquid oils such as N-lauroyl-L-glutamic acid di(phytosteryl/2-octyldodecyl) and the like.

The method for producing the gelatinous composition of the present invention is not particularly limited. For example, the target gelatinous composition can be obtained by heating and stirring a gelling agent composition until a homogeneous solution is formed, adding an oil agent, and mixing them under heating. Alternatively, the gelling agent composition and the oil agent may be mixed together under heating, or the oil agent may be heated and then the gelling agent composition may be added thereto and dissolved. The heating temperature varies depending on the types and contents of the gelling agent. The gelling agent composition can be obtained, for example, by mixing and heating the mixture at 99°C or lower using an oil bath or heater for 5 to 30 minutes, and then leaving same at room temperature.

The gelatinous composition of the present invention can also contain components generally used for cosmetic products such as various chelating agents, adiaphoretic active ingredient, surfactant, various additives, various powders and the like within the range where the effect of the present invention is not inhibited.

While various chelating agents are not particularly limited, preferable examples include chelating agents and a mixture thereof selected from the group consisting of triethylenetetramine, 2-thenoyltrifluoroacetone, thioglycolic acid, tartaric acid, succinic acid, 8-quinolinol, pyridine-2,6-dicarboxylic acid, pyridine, 1,10-tenanthroline, lactic acid, 8-hydroxyquinoline-5-sulfonic acid, glycine, 2,2'-pyridylethylenediamine, aurintricarboxylic acid, xylenol orange, 5-sulfosalicylic acid, salicylic acid, pyrocatechol-3,5-disulfonate, 4,5-dihydroxybenzene-1,3-disulfonic acid, 1,2-diaminocyclohexane-N,N,N',N'-tetraacetic acid, citric acid, oxalate, nitrilotriacetic acid, ethylenediamine-N,N,N',N'-tetraacetic acid, acetylacetone, and salts thereof.

The adiaphoretic active ingredient may be one selected from the group consisting of aluminum chlorohydrate, aluminum chloride, aluminum allantoin chlorohydrate, aluminum sulfate, zinc oxide, zinc paraphenolsulfonate, and zirconium aluminum complexes produced by reacting zirconyl chloride with aluminum hydroxide and aluminum chlorohydroxide, or a mixture thereof. As used herein, the adiaphoretic active ingredient refers to a component that suppresses the development of sweat by strong astringent action on the skin.

Examples of the surfactant include anionic surfactants such as N-long chain acyl amino acid salts (N-long chain acyl neutral amino acid salt and the like), N-long chain fatty acyl-N-methyltaurine salt, alkylsulfate and alkylene oxide adduct thereof, fatty acid amide ether sulfate, metal salt or weak base salt of fatty acid, sulfosuccinic acid-based surfactant, alkyl phosphate and alkylene oxide adduct thereof, alkylethercarboxylic acid, and the like; non-ionic surfactants such as ether type surfactants (glycerol ether and alkylene oxide adduct thereof and the like), etherester type surfactants (alkylene oxide adduct of glycerol ester, alkylene oxide adduct of sorbitan ester, polyoxyalkylene fatty acid ester and the like), ester type surfactants (glycerol fatty ester, fatty acid polyglycerol ester, sorbitan fatty ester, sucrose ester of fatty acid and the like), alkyl glucosides, nitrogen-containing non-ionic surfactants (hydrogenated castor oil pyroglutamic acid diester and ethylene oxide adduct thereof, fatty acid alkanol amide and the like); cationic surfactants such as quaternary fatty ammonium salts (alkyl ammonium chloride, dialkyl ammonium chloride and the like), aromatic quaternary ammonium salts (benzalkonium salt and the like), fatty acylarginine ester, and the like; as well as amphoteric surfactants such as betaine type surfactants (carboxybetaine and the like), aminocarboxylic acid type surfactants, imidazoline type surfactants, and the like, and silicone-based surfactants such as polyoxyethylene · methylpolysiloxane copolymer and the like.

Examples of various additives include amino acids such as glycine, alanine, serine, threonine, arginine, glutamic acid, aspartic acid, isoleucine, leucine, valine and the like; water-soluble polymer such as polyamino acid containing polyglutamic acid, polyaspartic acid and a salt thereof, gum arabic, alginates, xanthan gum, hyaluronic acid, hyaluronic acid salt, chitin, chitosan, water-soluble chitin, carboxyvinyl polymer, carboxymethyl cellulose, hydroxyethyl cellulose, hydroxypropyl trimethylammonium chloride, polychlorodimethylmethylene piperidium, polyvinylpyrrolidone derivative quaternary ammonium, cationized protein, collagen degradation products and a derivative thereof, acylated protein, and the like; sugar alcohol such as mannitol and alkylene oxide adduct thereof; lower alcohol such as ethanol, propanol and the like, animals and plants extract, nucleic acid, vitamin, enzyme, antiinflammatory agent, antimicrobial agent, preservative, antioxidant, UV absorber, pigment, dye, oxidation dye, pH adjuster, pearl agent, scattering agent such as zinc oxide, titanium oxide, and the like, wetting agent such as sodium pyrrolidonecarboxylate and the like, and the like.

Examples of the various powders include organic powders, for example, resin powders such as nylon beads, silicone beads and the like, nylon powder, metal fatty acid soap, yellow iron oxide, red iron oxide, black iron oxide, chrome oxide, cobalt oxide, carbon black, ultramarine blue, iron blue, zinc oxide, titanium oxide, zirconium oxide, silicon oxide, aluminum oxide, cerium oxide, micatitanium, boron nitride, barium sulfate, calcium carbonate, magnesium carbonate, aluminum silicate, magnesium silicate, silicon carbide, dye, lake, sericite, mica, talc, kaolin, plate-shaped barium sulfate, butterfly-shaped barium sulfate, titanium oxide fine particles, zinc oxide fine particles, iron oxide fine particles and the like, acylamino acids such as acyllysine, acylglutamic acid, acylarginine, acylglycine and the like, and the like, which may be further subjected to a surface treatment such as silicone treatment, fluorine compound treatment, silane coupling agent treatment, silanized organic titanate treatment, acylated lysine treatment, fatty acid treatment, metal soap treatment, oil treatment, amino acid treatment and the like.

The gelatinous composition of the present invention can be used as a cosmetic product, a perfumery, or a quasi-drug, either as it is or by incorporating the aforementioned components.

The gelatinous composition of the present invention can be used as it is as a wax substitute composition.

A wax substitute composition is a composition that does not contain a solid oil agent such as animal wax, vegetable wax, mineral wax, or synthetic wax but can produce a composition of the same quality as a final composition molded with wax.

Furthermore, the gelatinous composition of the present invention can be used as a base, texture improver, thickener, stabilizer, or gelling agent for cosmetic products, perfumery, pharmaceutical products, quasi-drugs, and the like. A base is a component among the raw materials of cosmetic products, and the like that is mainly used to give the product a shape, and is also called an excipient.

The shape of the cosmetic products, perfumery, pharmaceutical products and quasi-drugs containing the gelatinous composition of the present invention is not particularly limited, and examples thereof include paste, gel, cream, particulate, solid (stick (rod), sphere, sheet), and the like. Of these, stick and sphere are preferred, and stick is more preferred.

Specific examples of the cosmetic product, perfumery, and quasi-drug include solid cosmetic preparations such as adiaphoretics, lipsticks, lip rouge, sun protectants, solid foundations, concealers, makeup bases, cleansing agents and the like, gels or gel dispersions or emulsions such as facial cleansers, cleansing gels, milky lotions, massage creams, cold creams, moisture gels, facial masks, aftershave gels, milky foundations, blushes, mascaras, shampoos, rinses, hair growth agents, treatments, hair conditioners, tics, setting lotions, hair creams, hair waxes, hair mousses, perm solutions, hair dyes, hair colors, hair manicures, sun protectant oils, hand soaps, and the like, aromatics, adhesive preparations, and the like. Of these, solid cosmetics, particularly stick-shaped cosmetics, are preferred, and stick-shaped cosmetics such as adiaphoretics, lip rouge, lipsticks, creams, milky lotions, and sun protectants are preferred.

Specific examples of pharmaceutical products include external preparations such as ointments, creams, and gels, adhesive preparations, suppositories, and the like.

Cosmetic products, perfumery, pharmaceutical products, and quasi-drugs containing the gelatinous composition of the present invention can be manufactured according to conventional methods.

Specifically, the gelling agent composition of the present invention is included in the present invention as gelling agent composition 1 (also referred to as a high-concentration premix) of the present invention when it contains 20 to 40% by weight of (A) dibutyl lauroyl glutamide with respect to the total weight of the composition, and as gelling agent composition 2 (also referred to as a low-concentration premix) of the present invention when it contains 5 to 20% by weight of (A) dibutyl lauroyl glutamide with respect to the total weight of the composition.

The gelling agent composition 1 of the present invention is a gelling agent composition containing (A) to (C) and further (D), and containing, with respect to the total weight of the composition,
(A) dibutyl lauroyl glutamide: 20 to 40% by weight,
(B) an acyl acidic amino acid with an acyl group having 1 to 22 carbon atoms, or hydroxy acid: 1 to 22% by weight,
(C) a polar oil: 37 to 70% by weight, and
(D) a polyhydric alcohol: 5 to 20% by weight
wherein a weight ratio (B)/(A) is 0.03 to 0.7 and a weight ratio (D)/(A) is less than 0.5.

The content of each component in the gelling agent composition 1 of the present invention relative to the total weight of the composition and the ratio of each component are as follows.

From the aspect of gel strength, the content of (A) is generally 20% by weight or more, preferably 22% by weight or more, more preferably 25% by weight or more, further preferably 27% by weight or more, and the upper limit is 40% by weight or less, preferably 35% by weight or less, more preferably 33% by weight or less, relative to the total weight of the composition.

From the aspect of sweating suppression, the content of (B) is generally 1% by weight or more, preferably 2% by weight or more, more preferably 3% by weight or more, and the upper limit is 22% by weight or less, preferably 21% by weight or less, more preferably 20% by weight or less, relative to the total weight of the composition.

From the aspect of melting temperature, the content of (C) is generally 37% by weight or more, preferably 38% by weight or more, more preferably 40% by weight or more, and the upper limit is 70% by weight or less, preferably 65% by weight or less, more preferably 60% by weight or less, relative to the total weight of the composition.

From the aspects of sweating suppression and melting temperature, the content of (D) is generally 5% by weight or more, preferably 6% by weight or more, more preferably 7% by weight or more, further preferably 8% by weight or more, and the upper limit is 20% by weight or less, preferably 15% by weight or less, more preferably 12% by weight or less, further preferably 11% by weight or less, relative to the total weight of the composition.

From the aspect of sweating suppression, the weight ratio (B)/(A) is generally 0.03 or more, preferably 0.08 or more, more preferably 0.1 or more, and the upper limit is 0.7 or less, preferably 0.68 or less, more preferably 0.67 or less.

From the aspect of melting temperature, the weight ratio (C)/(A) is generally 1.2 or more, preferably 1.25 or more, more preferably 1.3 or more, and the upper limit is 2.5 or less, preferably 2 or less, more preferably 1.9 or less.

From the aspects of sweating suppression and melting temperature, the weight ratio (D)/(A) is generally 0.1 or more, preferably 0.2 or more, more preferably 0.25 or more, and the upper limit is less than 0.5, preferably 0.45 or less, more preferably 0.4 or less.

The preferred ranges of respective components in the gelling agent composition 1 of the present invention are as described above.

While the form of the gelling agent composition 1 of the present invention is not particularly limited, it can be provided as, for example, gel, particles, solid (rod-shape, sphere, sheet) or the like. Among these, gel or sphere is preferred.

The method for producing the gelling agent composition 1 of the present invention is not particularly limited. For example, the target gelling agent composition can be obtained by heating and stirring a mixture containing (A) to (D) until a homogeneous solution is formed, and then cooling the mixture. The heating temperature varies depending on the types and contents of (A) to (D). The gelling agent composition can be obtained, for example, by mixing and heating the mixture at 85 to 130°C using an oil bath or heater for 5 to 30 minutes, and then leaving same at room temperature.

In the present specification, the temperature at which a gelling agent composition that has become solid or gelled after standing is heated again to completely melt the composition is referred to as the "melting temperature".

The melting temperature of gelling agent composition 1 of the present invention is generally 99°C or less, preferably 98°C or less, more preferably 96°C or less, further preferably 95°C or less. Therefore, when used for producing cosmetic products and perfumery, a conventional treatment at a very high temperature is not necessary, cosmetic products and the like can be produced easily, and production steps of cosmetic products and the like can be simplified drastically.

The gel strength (gel rupture strength) of the gelling agent composition 1 of the present invention is generally 3000 g/cm² or more, preferably 5000 g/cm² or more, more preferably 6000 g/cm² or more, when measured at 25°C using, for example, a FUDOH rheometer (manufactured by Rheotec Co., Ltd.) or the like, a rod-shaped adapter having a diameter of 1.0 mm, under the conditions of insertion speed 6.0 cm/min, load of 200 g. The upper limit is generally 30000 g/cm² or less, preferably 25000 g/cm² or less, more preferably 21000 g/cm² or less.

The gel strength refers to the mechanical strength of an object that forms a gel, and is generally expressed as the force required to deform a gel with a certain shape or the force required to break the gel. It is mainly a measure of hardness. In the present specification, the gel strength is also referred to as the rupture strength or breaking strength of the gel, or simply strength. The breaking strength is also used as a measure of resistance to breakage when made into a stick shape.

As long as the gelling agent composition 1 of the present invention contains the above-mentioned (A) to (D) in the above-mentioned contents and ratios, it may contain the other additives already described, within a range that does not impair the effects of the present invention.

Since the gelling agent composition 1 of the present invention can impart optimal gel strength to solid cosmetics as described below, it can be used as a gelling agent composition for solid cosmetics, preferably for stick-shaped cosmetics.

A gelatinous composition obtained by adding the gelling agent composition 1 of the present invention to an oil agent (referred to as the gelatinous composition 1 of the present invention) is also included in the present invention.

From the aspects of obtaining a stable composition that has good texture, causes less sweating, can maintain a solid shape, and is not easily broken during use, the content of the gelling agent composition 1 of the present invention in the gelatinous composition 1 of the present invention is generally 5% by weight or more, preferably 10% by weight or more, more preferably 20% by weight or more, and the upper limit is generally 50% by weight or less, preferably 45% by weight or less, more preferably 40% by weight or less, relative to the total weight of gelatinous composition 1.

The gel strength (gel rupture strength) of the gelatinous composition 1 of the present invention is, from the aspects of resistance to breakage during use and a feeling of use, generally 1500 g/cm² or more, preferably 1600 g/cm² or more, more preferably 1700 g/cm² or more, when measured at 25°C using, for example, a FUDOH rheometer (manufactured by Rheotec Co., Ltd.) or the like, a spherical adapter having a diameter of 2.0 mm, under the conditions of insertion speed 6.0 cm/min, load of 200 g. The upper limit is generally 6600 g/cm² or less, preferably 6500 g/cm² or less, more preferably 6400 g/cm² or less.

Since the gelatinous composition 1 of the present invention has the above-mentioned rupture strength, it is applied to solid cosmetics and the like, and preferably applied to stick-shaped cosmetics.

When the gelatinous composition 1 of the present invention is molded into a stick-shaped cosmetic, the breaking strength is generally 100 g or more, preferably 105 g or more, more preferably 110 g or more, further preferably 120 g or more, when, for example, the maximum load required to break a stick-shaped composition (diameter 10 mm) at a load of 2 kg and an insertion speed of 30 cm/min was measured using a FUDOH rheometer (manufactured by Rheotec Corporation) or the like at 25°C. The upper limit is generally 400 g or less, preferably 350 g or less, more preferably 300 g or less.

The gelling agent composition 2 of the present invention is a gelling agent composition containing (A) to (C) and containing, with respect to the total amount of the composition
(A) dibutyl lauroyl glutamide: 5 to 20% by weight,
(B) an acyl acidic amino acid with an acyl group having 1 to 22 carbon atoms: 0.4 to 12% by weight, and
(C) a polar oil: 65 to 90% by weight,
wherein a weight ratio (B)/(A) is 0.03 to 0.7.

The content of each component in the gelling agent composition 2 of the present invention relative to the total weight of the composition and the ratio of each component are as follows.

From the aspect of gel strength, the content of (A) is generally 5% by weight or more, preferably 10% by weight or more, more preferably 15% by weight or more, and the upper limit is 20% by weight or less, preferably 19% by weight or less, more preferably 18% by weight or less, relative to the total weight of the composition.

From the aspect of suppression of sweating, the content of (B) is generally 0.4% by weight or more, preferably 0.5% by weight or more, more preferably 0.6% by weight or more, and the upper limit is 12% by weight or less, preferably 11% by weight or less, more preferably 10% by weight or less, relative to the total weight of the composition.

From the aspect of melting temperature, the content of (C) is generally 65% by weight or more, preferably 67% by weight or more, more preferably 70% by weight or more, and the upper limit is 90% by weight or less, preferably 85% by weight or less, more preferably 84.5% by weight or less, further preferably 84% by weight or less, relative to the total weight of the composition.

From the aspect of suppression of sweating, the weight ratio (B)/(A) is generally 0.03 or more, preferably 0.035 or more, more preferably 0.04 or more, and the upper limit is 0.7 or less, preferably 0.69 or less, more preferably 0.67 or less.

From the aspect of melting temperature, the weight ratio (C)/(A) is generally 3.6 or more, preferably 3.7 or more, more preferably 3.8 or more, and the upper limit is 6 or less, preferably 5.65 or less, more preferably 5.62 or less.

The gelling agent composition 2 of the present invention 2 may contain (D) in addition to (A) to (C). From the aspects of melting temperature and sweating suppression, the content of (D) is generally 0.01% by weight or more, preferably 0.03% by weight or more, more preferably 0.05% by weight or more, and the upper limit is 10% by weight or less, preferably 8% by weight or less, more preferably 6% by weight or less, relative to the total weight of the composition.

From the aspects of melting temperature and sweating suppression, the weight ratio (D)/(A) is generally 0.1 or more, preferably 0.2 or more, more preferably 0.3 or more, and the upper limit is less than 0.5, preferably 0.45 or less, more preferably 0.4 or less.

The preferred ranges of respective components in the gelling agent composition 2 of the present invention are as described above.

While the form of the gelling agent composition 2 of the present invention is not particularly limited, it can be provided as, for example, gel, particles, solid (rod-shape, sphere, sheet) or the like. Among these, gel or sphere is preferred.

The method for producing the gelling agent composition 2 of the present invention is not particularly limited. For example, the target gelatinous composition can be obtained by heating and stirring a mixture containing (A) to (C) or (A) to (D) until a homogeneous solution is formed, and then cooling the mixture. The heating temperature varies depending on the types and contents of (A) to (D). The gelling agent composition can be obtained, for example, by mixing and heating the mixture at 85 to 130°C using an oil bath or heater for 5 to 30 minutes, and then leaving same at room temperature.

The melting temperature of gelling agent composition 2 of the present invention is generally 100°C or less, preferably 96°C or less, more preferably 95°C or less, further preferably 94°C or less, particularly preferably 90°C or less. Therefore, when used for producing cosmetic products and perfumery, a conventional treatment at a very high temperature is not necessary, cosmetic products and the like can be produced easily, and production steps of cosmetic products and the like can be simplified drastically.

The gel strength (gel rupture strength) of the gelling agent composition 2 of the present invention is generally 2500 g/cm² or more, preferably 3000 g/cm² or more, more preferably 3400 g/cm² or more, when measured at 25°C using, for example, a FUDOH rheometer (manufactured by Rheotec Co., Ltd.) or the like, a rod-shaped adapter having a diameter of 1.0 mm, under the conditions of insertion speed 6.0 cm/min, load of 200 g. The upper limit is generally 5000 g/cm² or less, preferably 4500 g/cm² or less, more preferably 4300 g/cm² or less.

As long as the gelling agent composition 2 of the present invention contains the above-mentioned (A) to (D) or (A) to (D) in the above-mentioned contents and ratios, it may contain the other additives within a range that does not impair the effects of the present invention.

Since the gelling agent composition 2 of the present invention can impart optimal gel strength to solid cosmetics as described below, it can be used as a gelling agent composition for solid cosmetics, preferably for stick-shaped cosmetics.

A gelatinous composition 2 obtained by adding the gelling agent composition 2 of the present invention to an oil agent is also included in the present invention (also to be referred to as the gelatinous composition 2 of the present invention).

From the aspects of obtaining a stable composition that has good texture, causes less sweating, can maintain a solid shape, and is not easily broken during use, the content of the gelling agent composition 2 of the present invention in the gelatinous composition 2 of the present invention is generally 40% by weight or more, preferably 45% by weight or more, more preferably 50% by weight, and the upper limit is generally 75% by weight or less, preferably 70% by weight or less, more preferably 65% by weight or less, relative to the total weight of gelatinous composition 2.

The gel strength (gel rupture strength) of the gelatinous composition 2 of the present invention is, from the aspects of resistance to breakage during use and a feeling of use, generally 1500 g/cm² or more, preferably 1550 g/cm² or more, more preferably 1600 g/cm² or more, when measured at 25°C using, for example, a FUDOH rheometer (manufactured by Rheotec Co., Ltd.) or the like, a spherical adapter having a diameter of 2.0 mm, under the conditions of insertion speed 6.0 cm/min, load of 200 g. The upper limit is generally 6500 g/cm² or less, preferably 6400 g/cm² or less, more preferably 6300 g/cm² or less.

Since the gelatinous composition 2 of the present invention has the above-mentioned rupture strength, it is preferably applied to solid cosmetics and the like, and more preferably applied to stick-shaped cosmetics.

When the gelatinous composition 2 of the present invention is molded into a stick-shaped cosmetic, the breaking strength is generally 100 g or more, preferably 120 g or more, more preferably 130 g or more, when, for example, the maximum load required to break a stick-shaped composition (e.g., diameter 10 mm) at a load of 2 kg and an insertion speed of 30 cm/min was measured using a FUDOH rheometer (manufactured by Rheotec Corporation) or the like at 25°C. The upper limit is generally 400 g or less, preferably 350 g or less, more preferably 300 g or less.

As another embodiment of the present invention, a method for suppressing sweating in a solid cosmetic, including blending the above-mentioned gelling agent composition in an oil agent is also included.

The definition and preferred range of the gelling agent composition and the preferred range and blending ratio of the oil agent are also as described above.

Blending a gelling agent composition with an oil agent means combining (mixing) them to prepare a desired state, and means combining the gelling agent composition with an oil agent in accordance with the definition and preferred range of the above-mentioned gelling agent composition and the preferred range and blending ratio of the oil agent. The method for suppressing sweating of a solid cosmetic may include conventional steps such as a heating step, a stirring step, a subsequent cooling step, and the like of the blended mixture. The heating condition varies depending on the types and contents of the components contained in the gelling agent composition. For example, the mixture is mixed with heating at 85 to 130°C using an oil bath or heater for 5 to 30 minutes. The thus-obtained solid cosmetic suppresses sweating even after lapse of time.

The suppression of sweating can be evaluated by observing the "rising of oil", which is also called sweating, on the surface of the solid cosmetic.

As another embodiment of the present invention, a method for suppressing sweating in a solid cosmetic material, including blending the above-mentioned gelling agent composition 1 or 2 in an oil agent is also included.

The definition and preferred range of the gelling agent composition 1 or 2 and the preferred range and blending ratio of the oil agent are also as described above.

As another embodiment of the present invention, a thickener composition containing the following (A) to (D) is also included.

The thickener composition of the present invention contains, with respect to the total amount of the composition,
(A) dibutyl lauroyl glutamide: 20 to 40% by weight,
(B) an acyl acidic amino acid with an acyl group having 1 to 22 carbon atoms: 1 to 22% by weight,
(C) a polar oil: 37 to 70% by weight, and
(D) a polyhydric alcohol: 5 to 20% by weight
wherein a weight ratio (D)/(A) is less than 0.5.

A thickener means an additive used to give viscosity to cosmetic products and the like.

The content of each component in the thickener composition of the present invention relative to the total weight of the composition and the ratio of each component are as follows.

From the aspect of thickening effect, the content of (A) is generally 20% by weight or more, preferably 22% by weight or more, more preferably 25% by weight or more, further preferably 27% by weight or more, and the upper limit is 40% by weight or less, preferably 35% by weight or less, more preferably 33% by weight or less, relative to the total weight of the composition.

From the aspects of melting temperature and thickening effect, the content of (B) is generally 1% by weight or more, preferably 2% by weight or more, more preferably 3% by weight or more, and the upper limit is 22% by weight or less, preferably 21% by weight or less, more preferably 20% by weight or less, relative to the total weight of the composition.

From the aspect of melting temperature, the content of (C) is generally 37% by weight or more, preferably 38% by weight or more, more preferably 40% by weight or more, and the upper limit is 70% by weight or less, preferably 65% by weight or less, more preferably 60% by weight or less, relative to the total weight of the composition.

From the aspect of melting temperature, the content of (D) is generally 5% by weight or more, preferably 6% by weight or more, more preferably 7% by weight or more, further preferably 8% by weight or more, and the upper limit is 20% by weight or less, preferably 15% by weight or less, more preferably 12% by weight or less, further preferably 11% by weight or less, relative to the total weight of the composition.

The weight ratio (B)/(A) is generally 0.03 or more, preferably 0.08 or more, more preferably 0.1 or more, and the upper limit is 0.7 or less, preferably 0.68 or less, more preferably 0.67 or less.

From the aspect of melting temperature, the weight ratio (C)/(A) is generally 1.2 or more, preferably 1.25 or more, more preferably 1.3 or more, and the upper limit is 2.5 or less, preferably 2 or less, more preferably 1.9 or less.

From the aspect of melting temperature, the weight ratio (D)/(A) is generally 0.1 or more, preferably 0.2 or more, more preferably 0.25 or more, and the upper limit is less than 0.5, preferably 0.45 or less, more preferably 0.4 or less.

The definitions and preferred ranges of respective components in the thickener composition of the present invention are as described above.

While the form of the thickener composition of the present invention is not particularly limited, it can be provided as, for example, gel, particles, solid (rod-shape, sphere, sheet) or the like. Among these, gel or sphere is preferred.

The method for producing the thickener composition of the present invention is not particularly limited. For example, the target gelatinous composition can be obtained by heating and stirring a mixture containing (A) to (D) until a homogeneous solution is formed, and then cooling the mixture. The heating temperature varies depending on the types and contents of (A) to (D). The gelling agent composition can be obtained, for example, by mixing and heating the mixture at 85 to 130°C using an oil bath or heater for 5 to 30 minutes, and then leaving same at room temperature.

The melting temperature of thickener composition of the present invention is generally 99°C or less, preferably 98°C or less, more preferably 96°C or less, further preferably 95°C or less. Therefore, when used for producing cosmetic products and perfumery, a conventional treatment at a very high temperature is not necessary, cosmetic products and the like can be produced easily, and production steps of cosmetic products and the like can be simplified drastically.

As long as the thickener composition of the present invention contains the above-mentioned (A) to (D) in the above-mentioned contents and ratios, it may contain the other additives already described, within a range that does not impair the effects of the present invention.

Since the thickener composition of the present invention can impart optimal viscosity to cosmetics as described below, it can be used for emulsion cosmetics, preferably as a thickener composition for milky lotion.

An emulsion composition obtained by adding the thickener composition of the present invention to an oil agent and a solvent such as water (to be referred to as the emulsion composition of the present invention) is also included in the present invention.

The viscosity of the emulsion composition of the present invention may be varied as appropriate according to the use and temperature. A measurement method of viscosity includes a method for measuring the viscosity of the composition at 25°C using a Type B viscometer.

Since the emulsion composition of the present invention has an optimum viscosity for cosmetics, it is preferably used in emulsion cosmetics and the like, more preferably used in milky lotion.

### [Example]

The present invention is now explained in more detail by referring to Examples; however, the present invention is not limited to the following Examples.

### <Experimental Example 1: low concentration premix>

### Preparation of gelling agent composition and measurement of melting temperature

A mixture of (A)-(C) or (A)-(D) in the ratios shown in Tables 1-3 was heated and mixed until completely melted, and then allowed to cool to room temperature to obtain a gelling agent composition. The mixture was heated again, and the temperature at which it became transparent and completely melted was measured with a thermometer as the melting temperature.

### Preparation of stick

A mixture (40 to 60 g) of (A)-(C) or (A)-(D) in the ratios (% by weight) shown in Tables 1-3 was weighed into a glass bottle and melted in a water bath at 100°C or less until it became transparent. Other liquid oils were similarly heated, added to the mixture, and stirred until it became homogenous. When it became homogenous, a color paste was added, and when the color became uniform, the mixture was filled in a stick container.

### Evaluation of sweating

The stick was unrolled from the stick container and stored as was at -5°C for 2 hours, and then taken out into room temperature. One hour after taking out, the state of the stick surface was observed, and the level of oil rising (sweating) was evaluated on a three-point scale.
○: no oil droplets on surface
Δ: oil droplets were found in spots on surface
×: entire surface was oily and shiny

### Evaluation of texture

The stick formulation is applied to the palm of the hand at the base of the thumb and the texture is checked. A panel of one expert evaluated and judged the texture based on the following criteria:
○: good texture
Δ: not very good texture (poor adhesion)
×: bad texture (poor adhesion/rough)
××: very bad texture (poor adhesion/rough/severe oil smearing)

### Evaluation of breaking strength of stick formulation

With a stick (diameter 10 mm) unrolled, the maximum load when it was broken under conditions of 30 cm/min and load 2 kg was measured at 25°C using FUDOH rheometer (manufactured by Rheotec Corporation). It was determined that a load of 100 g or more, which is a level at which the stick does not break when used by unrolling, was determined to be preferable for stick-shaped cosmetics.

### Measurement of rupture strength of gelling agent

The gel strength (gel rupture strength) of the obtained each gelling agent composition was measured at 25°C using a FUDOH rheometer (manufactured by Rheotec Co., Ltd.) or the like, a rod-shaped adapter having a diameter of 1.0 mm, under the conditions of insertion speed 6.0 cm/min, load of 200 g.

### Measurement of rupture strength of gelatinous composition

The gel strength (gel rupture strength) of the obtained each gelatinous composition was measured at 25°C using a FUDOH rheometer (manufactured by Rheotec Co., Ltd.) or the like, a spherical adapter having a diameter of 2.0 mm, under the conditions of insertion speed 6.0 cm/min, load of 200 g.

The results are shown in Tables 1 to 3.

**[Table 1]**

| | | | | Comp. Ex. 1 | Ex. 1 | Ex. 2 | Comp. Ex. 2 | Ex. 3 | Comp. Ex. 3 | Comp. Ex. 4 | Comp. Ex. 5 | Ex. 4 | Ex. 5 | Ex. 6 | Ex. 7 |
|---|---|---|---|---|---|---|---|---|---|---|---|---|---|---|---|
| gelling agent composition | A | dibutyl lauroyl glutamide | | 9 | 9 | 9 | 9 | 9 | 8 | 9 | 9 | 9 | 9 | 9 | 9 |
| | | dibutyl ethylhexanoyl glutamide | | | | | | | | | | | | | |
| | B | stearoyl glutamic acid | | | | | - | 1.5 | 7.5 | - | 0.2 | 0.4 | 0.6 | 0.8 | 1 |
| | | lauroyl glutamic acid | | - | 3 | 6 | | | | | | | | | |
| | | cocoyl glutamic acid | | | | | | | | | | | | | |
| | C | octyldodecanol | | 51 | 48 | 45 | 51 | 49.5 | 44.5 | 51 | 50.8 | 50.6 | 50.4 | 50.2 | 50 |
| | | isostearyl alcohol | | | | | | | | | | | | | |
| | | hexyl decanol | | | | | | | | | | | | | |
| | | oleyl alcohol | | | | | | | | | | | | | |
| | D | 1,3-BG | | | | | | | | | | | | | |
| | | pentylene glycol | | | | | | | | | | | | | |
| E | | hydrogenated polyisobutene | | 25 | 25 | 25 | | | | | | | | | |
| | | liquid paraffin | | | | | | | | | | | | | |
| | | diisostearyl malate | | 15 | 15 | 15 | 15 | 15 | 15 | 15 | 15 | 15 | 15 | 15 | 15 |
| | | triethylhexanoin | | | | | | | | | | | | | |
| | | isotridecyl isononanoate | | | | | 20 | 20 | 20 | 20 | 20 | 20 | 20 | 20 | 20 |
| | | α olefin oligomer | | | | | 5 | 5 | 5 | 5 | 5 | 5 | 5 | 5 | 5 |
| base total | | | | 100 | 100 | 100 | 100 | 100 | 100 | 100 | 100 | 100 | 100 | 100 | 100 |
| gelling agent composition concentration in gelatinous composition | | | | 60 | 60 | 60 | 60 | 60 | 60 | 60 | 60 | 60 | 60 | 60 | 60 |
| ratio in gelling agent composition | ratio of (A) | 15 | 15 | 15 | 15 | 15 | 13.3333 | 15 | 15 | 15 | 15 | 15 | 15 | | |
| | ratio of (B) | 0 | 5 | 10 | 0 | 2.5 | 12.5 | 0 | 0.33333 | 0.66667 | 1 | 1.33333 | 1.66667 | | |
| | ratio of (C) | 85 | 80 | 75 | 85 | 82.5 | 74.1667 | 85 | 84.6667 | 84.3333 | 84 | 83.6667 | 83.3333 | | |
| | ratio of (D) | 0 | 0 | 0 | 0 | 0 | 0 | 0 | 0 | 0 | 0 | 0 | 0 | | |
| | (D) / (A) | 0.00 | 0.00 | 0.00 | 0.00 | 0.00 | 0.00 | 0.00 | 0.00 | 0.00 | 0.00 | 0.00 | 0.00 | | |
| | (B) / (A) | 0.00 | 0.33 | 0.67 | 0.00 | 0.17 | 0.94 | 0.00 | 0.02 | 0.04 | 0.07 | 0.09 | 0.11 | | |
| | (C) / (A) | 5.67 | 5.33 | 5.00 | 5.67 | 5.50 | 5.56 | 5.67 | 5.64 | 5.62 | 5.60 | 5.58 | 5.56 | | |
| evaluation of gelling agent composition | melting temperature (°C) | 102 | 96 | 90 | 100 | 98 | 98 | 102 | 100 | 100 | 100 | 100 | 100 | | |
| | gel strength (g/cm2) | 4143.11 | 3722.94 | 3603.26 | 2537.56 | not tested | not tested | not tested | not tested | not tested | not tested | not tested | 3693.66 | | |
| evaluation of gelatinous composition | sweating | × | ○ | ○ | × | ○ | Δ | × | Δ | ○ | ○ | ○ | ○ | | |
| | texture | Δ | ○ | ○ | Δ | ○ | ○ | Δ | ○ | ○ | ○ | ○ | ○ | | |
| | breaking strength (g) of stick | 247.19 | 153.39 | 142.38 | 224.76 | not tested | not tested | not tested | 161.52 | 139.9 | 153.45 | 150.26 | 155.14 | | |
| | gel strength (g/cm2) | 5407.45 | 3236.58 | 1792.4 | 3920.94 | 3425.02 | 6184.77 | 4505.36 | 3062.46 | 3097.79 | 3285.92 | 3156.05 | 3511.6 | | |

**[Table 2]**

| | | | | Comp. Ex. 6 | Comp. Ex. 7 | Ex. 8 | Ex. 9 | Ex. 10 | Comp. Ex. 8 | Comp. Ex. 9 | Comp. Ex. 10 | Comp. Ex. 11 | Comp. Ex. 12 | Ex. 11 | Ex. 12 | Ex. 13 | Ex. 14 | Ex. 15 | **Ex.** 16 |
|---|---|---|---|---|---|---|---|---|---|---|---|---|---|---|---|---|---|---|---|
| gelling agent composition | A | dibutyl lauroyl glutamide | | 9 | 9 | 9 | 9 | 9 | 9 | 9 | 9 | 9 | 9 | 9 | 9 | 9 | 9 | 9 | 9 |
| | | dibutyl ethylhexanoyl glutamide | | | | | | | | | | | | | | | | | |
| | B | stearoyl glutamic acid | | - | - | 3 | 3 | 3 | 3 | 3 | | | | | | | | | |
| | | lauroyl glutamic acid | | | | | | - | - | - | | | | 3 | 3 | 3 | 3 | 3 | 3 |
| | | cocoyl glutamic acid | | | | | | | | | | | | | | | | | |
| | C | octyldodecanol | | 48 | 48 | 45 | 45 | 35 | 32 | 29 | | | | | | | | | |
| | | isostearyl alcohol | | | | | | | | | 51 | | | 48 | | | 45 | | |
| | | hexyldecanol | | | | | | | | | | 51 | | | 48 | | | 45 | |
| | | oleyl alcohol | | | | | | | | | | | 51 | | | 48 | | | 45 |
| | D | 1,3-BG | | 3 | 3 | 3 | 3 | 3 | 6 | 9 | | | | | | | 3 | 3 | 3 |
| | | pentylene glycol | | | | | | | | | | | | | | | | | |
| E | | hydrogenated polyisobutene | | | | | | | | | 25 | 25 | 25 | 25 | 25 | 25 | 25 | 25 | 25 |
| | | liquid parafin | | 40 | | 40 | | 50 | 50 | 50 | | | | | | | | | |
| | | diisostearyl malate | | | | | | | | | 15 | 15 | 15 | 15 | 15 | 15 | 15 | 15 | 15 |
| | | triethylhexanoin | | | 40 | | 40 | | | | | | | | | | | | |
| | | isotridecyl isononanoate | | | | | | | | | | | | | | | | | |
| | | α olefin oligomer | | | | | | | | | | | | | | | | | |
| base total | | | 100 | | 100 | 100 | 100 | 100 | 100 | 100 | 100 | 100 | 100 | 100 | 100 | 100 | 100 | 100 | 100 |
| gelling agent composition concentration in gelatinous composition | | | 60 | | 60 | 60 | 60 | 50 | 50 | 50 | 60 | 60 | 60 | 60 | 60 | 60 | 60 | 60 | 60 |
| ratio in gelling agent composition | ratio of (A) | | 15 | | 15 | 15 | 15 | 18 | 18 | 18 | 15 | 15 | 15 | 15 | 15 | 15 | 15 | 15 | 15 |
| | ratio of (B) | | 0 | | 0 | 5 | 5 | 6 | 6 | 6 | 0 | 0 | 0 | 5 | 5 | 5 | 5 | 5 | 5 |
| | ratio of (C) | | 80 | | 80 | 75 | 75 | 70 | 64 | 58 | 85 | 85 | 85 | 80 | 80 | 80 | 75 | 75 | 75 |
| | ratio of (D) | | 5 | | 5 | 5 | 5 | 6 | 12 | 18 | 0 | 0 | 0 | 0 | 0 | 0 | 5 | 5 | 5 |
| | (D) / (A) | | 0.33 | | 0.33 | 0.33 | 0.33 | 0.33 | 0.67 | 1.00 | 0.00 | 0.00 | 0.00 | 0.00 | 0.00 | 0.00 | 0.33 | 0.33 | 0.33 |
| | (B) / (A) | | 0.00 | | 0.00 | 0.33 | 0.33 | 0.33 | 0.33 | 0.33 | 0.00 | 0.00 | 0.00 | 0.33 | 0.33 | 0.33 | 0.33 | 0.33 | 0.33 |
| | (C) / (A) | | 5.33 | | 5.33 | 5.00 | 5.00 | 3.89 | 3.56 | 3.22 | 5.67 | 5.67 | 5.67 | 5.33 | 5.33 | 5.33 | 5.00 | 5.00 | 5.00 |
| evaluation of gelling agent composition | melting temperature (°C) | | 96 | | 96 | <90 | <90 | 94 | <92 | <92 | 96 | 94 | 96 | <94 | <94 | <94 | <94 | <94 | <94 |
| | gel strength (g/cm2) | | 4129. 11 | | not tested | 3454. 29 | not tested | 4257.7 | 3570. 16 | 3607. 08 | not tested | not tested | not tested | not tested | not tested | not tested | not tested | not tested | not tested |
| evaluation of gelatinous composition | sweating | | × | | × | ○ | ○ | ○ | Δ | × | × | × | × | ○ | ○ | ○ | ○ | ○ | ○ |
| | texture | | not tested | | not tested | ○ | ○ | ○ | ○ | Δ | not tested | not tested | not tested | not tested | not tested | not tested | not tested | not tested | not tested |
| | breaking strength (g) of stick | | 146. 91 | | 115.7 | 172. 06 | 176. 44 | 249. 08 | 189. 72 | 190. 18 | 196. 01 | 157. 56 | 206 | 139. 52 | 143. 68 | 188. 09 | 259. 27 | 258. 19 | 261. 51 |
| | gel strength (g/cm2) | | 3870. 01 | | 3961. 69 | 6237. 29 | 3811. 13 | 2868. 61 | 2340. 53 | 2246. 31 | 2850. 15 | 2873. 39 | 2873. 39 | 2268. 28 | 1868. 48 | 2530. 57 | 1837. 6 | 1635. 48 | 1956. 65 |

**[Table 3]**

| | | | Comp. Ex. 13 | Comp. Ex. 14 | Comp. Ex. 15 | Comp. Ex. 16 |
|---|---|---|---|---|---|---|
| gelling agent composition | A | dibutyl lauroyl glutamide | 10 | 7.5 | 8 | 7.5 |
| | | dibutyl ethylhexanoyl glutamide | | 5 | 2 | 5 |
| | B | stearoyl glutamic acid | | | | |
| | | lauroyl glutamic acid | | | | |
| | | cocoyl glutamic acid | | | 0.25 | 0.5 |
| | C | octyldodecanol | | | | |
| | | isostearyl alcohol | 5 | 30 | 10 | 30 |
| | | hexyldecanol | | | | |
| | | oleyl alcohol | | | | |
| | D | 1,3-BG | | | | |
| | | pentylene glycol | 35 | 7.5 | 29.75 | 7 |
| E | | hydrogenated polyisobutene | | | | |
| | | liquid paraffin | 50 | 50 | 50 | 50 |
| | | diisostearyl malate | | | | |
| | | triethylhexanoin | | | | |
| | | isotridecyl isononanoate | | | | |
| | | α olefin oligomer | | | | |
| | | isopropyl myristate | | | | |
| | | paste color | | | | |
| base total | | | 100 | 100 | 100 | 100 |
| gelling agent composition concentration in gelatinous composition | | | 50 | 50 | 50 | 50 |
| ratio in gelling agent composition | | ratio of (A) | 20 | 25 | 20 | 25 |
| | | ratio of (B) | 0 | 0 | 0.5 | 1 |
| | | ratio of (C) | 10 | 60 | 20 | 60 |
| | | ratio of (D) | 70 | 15 | 59.5 | 14 |
| | | (D) / (A) | 3.50 | 0.60 | 2.98 | 0.56 |
| | | (B) / (A) | 0.00 | 0.00 | 0.03 | 0.04 |
| | | (C) / (A) | 0.50 | 2.40 | 1.00 | 2.40 |
| evaluation of gelling agent composition | | melting temperature (°C) | 76.5 | 76 | 57.5 | 73.5 |
| | | gel strength (g/cm2) | 3610.9 | 10067.48 | 6433.66 | 10681.18 |
| evaluation of gelatinous composition | | sweating | × | × | × | × |
| | | texture | × | ○ | × | ○ |
| | | breaking strength (g) of stick | 3.18 | 295.7 | 15.86 | 341.27 |
| | | gel strength (g/cm2) | separation | 3442.576 | separation | 4282.717 |

As shown in Tables 1 to 3, when (D) is blended at 12% or more, suppression of sweating is lost. Furthermore, when the (B)/(A) ratio is too large, the sweating suppression effect of (B) is weakened and the texture also becomes poor.

### <Experimental Example 2>

### <Experimental Example 2: low concentration premix>

### Preparation of gelling agent composition and measurement of melting temperature

A mixture of (A)-(D) in the ratio shown in Table 4 was heated and mixed until completely melted, and then allowed to cool to room temperature to obtain a gelling agent composition. The mixture was heated again, and the temperature at which it became transparent was measured with a thermometer as the temperature of complete melting (melting temperature).

### Preparation of stick

A mixture (60 g) of (A)-(D) in the ratio shown in Table 4 was weighed into a glass bottle and melted in a water bath at 100°C or less until it became transparent. Other liquid oils were similarly heated, added to the mixture, and stirred until it became homogenous. When it became homogenous, a color paste was added, and when the color became uniform, the mixture was filled in a stick container.

### Evaluation of sweating

Performed in the same manner as in Experimental Example 1.

### Evaluation of gel strength (breaking strength) of stick formulation

With a stick (diameter 10 mm) unrolled, the maximum load when it was broken under conditions of 30 cm/min and load 2 kg was measured at 25°C using FUDOH rheometer (manufactured by Rheotec Corporation). It was determined that a load of 100 g or more, which is a level at which the stick does not break when used by unrolling, was determined to be preferable for stick-shaped cosmetics.

**[Table 4]**

| | | | hydrocarbon oil | ester oil | amino acid oil | trigly oil |
|---|---|---|---|---|---|---|
| mineral oil | | | 40 | | | |
| isotridecyl isononanoate | | | | 40 | | |
| N-lauroyl-L-glutamic acid di(phytosteryl/2-octyldodecyl) | | | | | 40 | |
| triethylhexanoin | | | | | | 40 |
| gelling agent composition of Ex. 8 | A | dibutyl lauroyl glutamide | 9.0 | | | |
| | B | stearoyl glutamic acid | 3.0 | | | |
| | C | octyldodecanol | 45.0 | | | |
| | D | butylene glycol | 3.0 | | | |
| base total | | | 100 | 100 | 100 | 100 |
| melting temperature of gelling agent composition | | | <90°C | | | |
| sweating of gelatinous composition | | | ○ | ○ | ○ | O |
| breaking strength (g) of stick | | | 172.06 | 176.44 | 334.27 | 158.61 |

As shown in Table 4, by using N-lauroyl-L-glutamic acid phytosterol ester oil as (E) oil agent, the breaking strength of the gelatinous composition is improved.

### <Experimental Example 3: high concentration premix>

### Preparation of gelling agent and measurement of melting temperature

A mixture of (A)-(D) in the ratio shown in Table 5 was heated and mixed until completely melted, and then allowed to cool to room temperature to obtain a gelling agent composition. The mixture was heated again, and the temperature at which it became transparent was measured with a thermometer as the temperature of complete melting (melting temperature). The same procedure was performed with existing oil gelling agents.

### Preparation of stick

A mixture (27-30 g) of (A)-(D) in the ratio shown in Table 5 was weighed into a glass bottle and melted in a water bath at 96°C or less until it became transparent. Other liquid oils were similarly heated, added to the mixture, and stirred until it became homogenous. When it became homogenous, a color paste was added, and when the color became uniform, the mixture was filled in a stick container. Similarly, sticks were prepared using existing oil gelling agents in the ratios shown in Table 6.

### Evaluation of sweating

Performed in the same manner as in Experimental Example 1.

### Evaluation of breaking strength of stick formulation

With a stick (diameter 10 mm) unrolled, the maximum load when it was broken under conditions of 30 cm/min and load 2 kg was measured at 25°C using FUDOH rheometer (manufactured by Rheotec Corporation). It was determined that a load of 100 g or more, which is a level at which the stick does not break when used by unrolling, was determined to be preferable for stick-shaped cosmetics.

Various evaluations were performed based on the same criteria as in Experimental Example 1, and the results are shown in Tables 5 and 6.

**[Table 5]**

| | | | Ex. 17 | Ex. 18 | Ex. 19 | Ex. 20 | Ex. 21 | Comp. Ex. 17 | Ex. 22 | Ex. 23 | Ex. 24 | Ex. 25 | Ex. 26 | Ex. 27 | Ref . Ex. 1 | Ex. 28 | Comp. Ex. 18 |
|---|---|---|---|---|---|---|---|---|---|---|---|---|---|---|---|---|---|
| gelling agent composition | A | dibutyl lauroyl glutamide | 9 | 9 | 9 | 9 | 9 | 9 | 9 | 9 | 9 | 9 | 9 | 9 | 9 | 9 | 9 |
| | | ethylhexyl lauroyl glutamide | | - | - | - | - | | - | - | | | - | - | | | |
| | B | stearoyl glutamic acid | 1 | 3 | 4 | 5 | 6 | 7 | - | - | | | 5 | 5 | 5 | | |
| | | lauroyl glutamic acid | | - | - | - | - | | 3 | 6 | | | - | - | | | |
| | | cocoyl glutamic acid | | | | | | | | | | | | | | 3 | |
| | | lactic acid | | | | | | | | | 2 | | | | | | |
| | | citric acid | | | | | | | | | | 2 | | | | | |
| | C | isostearic acid | 17 | 15 | 14 | 13 | 12 | 11 | 15 | 12 | 16 | 16 | 13 | 13 | 16 | 15 | 15 |
| | D | 1,3-BG | 3 | 3 | 3 | 3 | 3 | 3 | 3 | 3 | 3 | 3 | | | | 3 | 3 |
| | | pentylene glycol | | | | | | | | | | | 3 | | | | |
| | | propylene glycol | | - | - | - | - | | - | - | | | | 3 | | | |
| E | | lauroyl glutamic acid di | | | | | | | | | | | | | | 20 | 20 |
| | | hydrogenated polyisobutene | 17 | 17 | 17 | 17 | 17 | 17 | 17 | 17 | 17 | 17 | 17 | 17 | 17 | | |
| | | diisostearyl malate | 17 | 17 | 17 | 17 | 17 | 17 | 17 | 17 | 17 | 17 | 17 | 17 | 17 | 20 | 20 |
| | | triethylhexanoin | 17 | 17 | 17 | 17 | 17 | 17 | 17 | 17 | 17 | 17 | 17 | 17 | 17 | 20 | 20 |
| | | isopropyl myristate | 17 | 17 | 17 | 17 | 17 | 17 | 17 | 17 | 17 | 17 | 17 | 17 | 17 | | |
| | | paste color | 2 | 2 | 2 | 2 | 2 | 2 | 2 | 2 | 2 | 2 | 2 | 2 | 2 | 10 | 10 |
| base total | | | 100 | 100 | 100 | 100 | 100 | 100 | 100 | 100 | 100 | 100 | 100 | 100 | 100 | 100 | 97 |
| gelling agent composition concentration in gelatinous composition | | | 30 | 30 | 30 | 30 | 30 | 30 | 30 | 30 | 30 | 30 | 30 | 30 | 30 | 30 | 27 |
| ratio in gelling agent composition | ratio of (A) | | 30 | 30 | 30 | 30 | 30 | 30 | 30 | 30 | 30 | 30 | 30 | 30 | 30 | 30 | 33. 33333 |
| | ratio of (B) | | 3.33 | 10.00 | 13.33 | 16.67 | 20.00 | 23.33 | 10.00 | 20.00 | 6.67 | 6.67 | 16.67 | 16.67 | 16.67 | 10.00 | 0.00 |
| | ratio of (C) | | 56.67 | 50.00 | 46.67 | 43.33 | 40.00 | 36.67 | 50.00 | 40.00 | 53.33 | 53.33 | 43.33 | 43.33 | 53.33 | 50.00 | 55.56 |
| | ratio of (D) | | 10 | 10 | 10 | 10 | 10 | 10 | 10 | 10 | 10 | 10 | 10 | 10 | 0 | 10 | 11. 11111 |
| | (D) / (A) | | 0.33 | 0.33 | 0.33 | 0.33 | 0.33 | 0.33 | 0.33 | 0.33 | 0.33 | 0.33 | 0.33 | 0.33 | 0.00 | 0.33 | 0.33 |
| | (B) / (A) | | 0.11 | 0.33 | 0.44 | 0.56 | 0.67 | 0.78 | 0.33 | 0.67 | 0.22 | 0.22 | 0.56 | 0.56 | 0.56 | 0.33 | 0.00 |
| | (C) / (A) | | 1.89 | 1.67 | 1.56 | 1.44 | 1.33 | 1.22 | 1.67 | 1.33 | 1.78 | 1.78 | 1.44 | 1.44 | 1.78 | 1.67 | 1.67 |
| evaluation of gelling agent composition | melting temperature (°C) | | 95 | 94 | 94 | 94 | 94 | 94 | 92 | 92 | 90 | 90 | 94 | 92 | 102 | 95 | 102 |
| | gel strength (g/cm2) | | 8239. 11 | not tested | not tested | not tested | 20967. 66 | not tested | 8618. 54 | not tested | 6291. 06 | 10813.6 | not tested | 19354. 47 | not tested | not tested | not tested |
| evaluation of gelatinous composition | sweating | | ○ | ○ | ○ | O | ○ | Δ | ○ | ○ | Δ | O | ○ | ○ | × | ○ | Δ |
| | texture | | ○ | ○ | ○ | ○ | ○ | Δ | ○ | ○ | ○ | O | ○ | ○ | ○ | ○ | ○ |
| | breaking strength (g) of stick | | 125. 52 | 197. 23 | 180.8 | 225. 48 | 240. 18 | 238. 22 | 125. 24 | 160.6 | 113. 33 | 117. 37 | 281. 03 | 222. 95 | 336. 88 | 228. 47 | 267. 91 |
| | gel strength (g/cm2) | | 1763. 12 | 5385. 17 | 5720. 67 | 5323.1 | 5836. 22 | 3026. 811 | 2815. 45 | 5713. 67 | 2141. 591 | 2256. 501 | 6373. 21 | 5071 | 5018. 796 | 3824.5 | 4117. 66 |

**[Table 6]**

| | | Comp. Ex. 19 | Comp. Ex. 20 | Comp. Ex. 21 |
|---|---|---|---|---|
| existing oil gelling agent | AN-GEL | 30 | | |
| | dextrin palmitate | | 20 | |
| | 12-hydroxystearic acid | | | 10 |
| others | hydrogenated polyisobutene | 17 | 20 | 22 |
| | diisostearyl malate | 17 | 20 | 22 |
| | triethylhexanoin | 17 | 19 | 22 |
| | isopropyl myristate | 17 | 19 | 22 |
| | paste color | 2 | 2 | 2 |
| base total | | 100 | 100 | 100 |
| evaluation of gelling agent composition | melting temperature (°C) | 75 | 84 | 84 |
| | gel strength (g/cm2) | not tested | not tested | not tested |
| evaluation of gelatinous composition | sweating | × × | ○ | × |
| | texture | ○ | × | Δ |
| | breaking strength (g) of stick | 287.55 | 60.96 | 268.2 |
| | gel strength (g/cm2) | - | - | - |

As shown in Tables 5 and 6, when the (B)/(A) ratio is too high, the sweating suppression effect of (B) is weakened and the texture becomes poor. When (D) is included, the texture becomes desirable and the melting temperature becomes lower, making it easier to melt in a steam oven.

### <Experimental Example 4: high concentration premix>

### Preparation of milky lotion

Milky lotion (W/O) is prepared according to the ratio (% by weight) shown in Table 7.
(1) a described in Table 7 is stirred well
(2) b is gradually added while stirring a at 90°C
(3) c is gradually added while stirring a at 80°C
(4) d is at 80°C and added to a while stirring
(5) mixture is cooled to room temperature while stirring

### Measurement of viscosity

The viscosity of the obtained each milky lotion sample was measured using a Type B viscometer TVB-10 ((Toki Sangyo) and rotor No.3 under the conditions of 12 rpm, rpm time 30 sec.

### Observation of separation state

Milky lotion samples were placed at room temperature for 24 hours and the separation state was visually observed.
○: no separation was observed
×: separation was observed

**[Table 7]**

| | | | | Comp. Ex. 22 | Ex. 29 |
|---|---|---|---|---|---|
| a | | cyclohexasiloxane | | 30.2 | 31.7 |
| | | PEG-9 dimethicone | | 2.0 | 2.0 |
| | | dimethicone | | 3.0 | 3.0 |
| | | squalane | | 2.0 | 2.0 |
| b | | GP-1 | | - | - |
| | | BG | | 5.0 | - |
| | premix | A | dibutyl lauroyl glutamide | - | 1.05 |
| | | B | cocoyl glutamic acid | - | 0.35 |
| | | C | isostearic acid | - | 1.75 |
| | | D | butylene glycol | - | 0.35 |
| c | | zinc oxide | | 8.0 | 8.0 |
| | | titanium oxide | | 5.0 | 5.0 |
| d | | antiseptic | | appropriate amount | appropriate amount |
| | | sodium pyrrolidonecarboxylate solution | | 0.5 | 0.5 |
| | | water | | 44.30 | 44.30 |
| total | | | | 100.0 | 100.0 |
| viscosity (Pa·s) | | | | 2.38 | 7.41 |
| separation state | | | | × | ○ |

The compounds used are as follows:
dibutyl lauroyl glutamide: GP-1 (Ajinomoto Co., Ltd.)
dibutyl ethylhexanoyl glutamide: EB-21 (Ajinomoto Co., Ltd.)
stearoyl glutamic acid: Amisoft (registered trademark) HA-P (Ajinomoto Co., Ltd.)
lauroyl glutamic acid: Amisoft (registered trademark) LA-D (Ajinomoto Co., Ltd.)
octyl dodecanol: Lisonol 20SP
isostearyl alcohol: isostearyl alcohol EX
hexyldecanol: Lisonol 16SP
oleyl alcohol: oleyl alcohol
hydrogenated polyisobutene: Pearleem 18
diisostearyl malate: Cosmol 222
triethylhexanoin: T.I.O (Kyukyu Alcohol Kogyo Co., Ltd.) isopropyl myristate: IPM-R
mineral oil: P-55
isotridecyl isononanoate: Salacos 913 (Nisshin Oillio Group, Inc.)
N-Lauroyl-L-glutamic acid di(phytosteryl/2-octyldodecyl): Eldew PS-203 (Ajinomoto Co., Inc.)
dimethicone: KF-56A-10Cs (Shin-Etsu Chemical Co., Ltd.)
dimethicone: SH200c 350cs (Dow Chemical Co., Ltd.)
butylene glycol: 1,3-BG (Daicel Corporation)
cyclohexasiloxane: DC246 (Dow Chemical Co., Ltd.)
PEG-9 dimethicone: KF-6017 (Shin-Etsu Chemical Co., Ltd.)
cocoyl glutamic acid: Amisoft CA (Ajinomoto Co., Inc.)
isostearic acid: isostearic acid EX (Kyukyu Alcohol Kogyo Co., Ltd.)
zinc oxide: MZY-303S (Teika Co., Ltd.)
titanium oxide: MT-100TV (Teika Co., Ltd.)
DL-pyrrolidone carboxylate sodium solution (PCA-Na water): AJIDEW NL-50 (Ajinomoto Co., Ltd.)

### [Industrial Applicability]

By using the gelling agent composition of the present invention, a wide variety of cosmetic products and the like that are superior in texture and moldability and have good stability can be easily produced without damaging the components blended in cosmetic products, perfumery, quasi-drugs, and the like.

This application is based on a patent application No. filed in Japan 2022-086341, the contents of which are encompassed in full herein.

## Claims

1. A gelling agent composition comprising the following (A) to (C) with respect to the total amount of the composition
(A) dibutyl lauroyl glutamide: 5 to 40% by weight,
(B) an acyl acidic amino acid with an acyl group having 1 to 22 carbon atoms, or hydroxy acid: 0.4 to 22% by weight, and
(C) a polar oil: 37 to 90% by weight,
wherein a weight ratio (B)/(A) is 0.03 to 0.7.

2. The gelling agent composition according to claim 1, further comprising (D) a polyhydric alcohol and comprising, with respect to the total amount of the composition,
(A) dibutyl lauroyl glutamide: 20 to 40% by weight,
(B) an acyl acidic amino acid with an acyl group having 1 to 22 carbon atoms, or hydroxy acid: 1 to 22% by weight,
(C) a polar oil: 37 to 70% by weight, and
(D) a polyhydric alcohol: 5 to 20% by weight
wherein a weight ratio (D)/(A) is less than 0.5.

3. The gelling agent composition according to claim 2, wherein the (B) acidic amino acid is glutamic acid.

4. The gelling agent composition according to claim 2, wherein the (B) acyl acidic amino acid is at least one type selected from the group consisting of N-lauroyl glutamic acid, N-myristoyl glutamic acid, N-palmitoyl glutamic acid, N-stearoyl glutamic acid, and N-cocoyl glutamic acid.

5. The gelling agent composition according to claim 2, wherein the (B) hydroxy acid is at least one type selected from the group consisting of lactic acid, citric acid, and malic acid.

6. The gelling agent composition according to claim 2, wherein the (C) polar oil is at least one type selected from a fatty acid having 8 to 22 carbon atoms and a higher alcohol having 8 to 24 carbon atoms.

7. The gelling agent composition according to claim 6, wherein the (C) polar oil is at least one type selected from isostearyl alcohol, 2-hexyldecanol, 2-octyldodecanol, oleyl alcohol, 2-decyltetradecanol, isostearic acid, and stearic acid.

8. The gelling agent composition according to claim 2, wherein the weight ratio (D)/(A) is 0.1 to 0.45.

9. The gelling agent composition according to claim 2, wherein the (D) polyhydric alcohol is a polyhydric alcohol having 2 to 12 carbon atoms.

10. The gelling agent composition according to claim 2, wherein the (D) polyhydric alcohol is at least one type selected from the group consisting of butylene glycol, propylene glycol, propanediol, pentylene glycol, isopentyldiol, hexanediol, and dipropylene glycol.

11. The gelling agent composition according to claim 2, which is used for a solid cosmetic.

12. The gelling agent composition according to claim 11, wherein the solid cosmetic is a stick-shaped cosmetic.

13. The gelling agent composition according to claim 2, which melts at 99°C or lower.

14. A gelatinous composition comprising the gelling agent composition according to claim 2 and an oil agent.

15. The gelatinous composition according to claim 14, comprising 5 to 50% by weight of the gelling agent composition relative to the total weight of the gelatinous composition.

16. The gelatinous composition according to claim 14, having a rupture strength of 1500 g/cm² or more and 6600 g/cm² or less as measured at an insertion speed of 6 cm/min.

17. The gelatinous composition according to claim 14, which is a stick-shaped cosmetic.

18. The gelling agent composition according to claim 1, comprising, with respect to the total amount of the composition,
(A) dibutyl lauroyl glutamide: 5 to 20% by weight,
(B) an acyl acidic amino acid with an acyl group having 1 to 22 carbon atoms: 0.4 to 12% by weight, and
(C) a polar oil: 65 to 90% by weight
wherein a weight ratio (B)/(A) is 0.03 to 0.7.

19. The gelling agent composition according to claim 18, wherein the (B) acidic amino acid is glutamic acid.

20. The gelling agent composition according to claim 18, wherein the (B) acyl acidic amino acid is at least one type selected from the group consisting of N-lauroyl glutamic acid, N-myristoyl glutamic acid, N-palmitoyl glutamic acid, N-stearoyl glutamic acid, and N-cocoyl glutamic acid.

21. The gelling agent composition according to claim 18, wherein the (C) polar oil is at least one type selected from a fatty acid having 8 to 22 carbon atoms and a higher alcohol having 8 to 24 carbon atoms.

22. The gelling agent composition according to claim 21, wherein the (C) polar oil is at least one selected from isostearyl alcohol, 2-hexyldecanol, 2-octyldodecanol, oleyl alcohol, 2-decyltetradecanol, isostearic acid, and stearic acid.

23. The gelling agent composition according to claim 18, further comprising (D) a polyhydric alcohol.

24. The gelling agent according to claim 23, wherein a weight ratio (D)/(A) is less than 0.5.

25. The gelling agent according to claim 23, wherein the (D) polyhydric alcohol is contained at 0.01 to 10% by weight with respect to the total amount of the composition and a weight ratio (D)/(A) is 0.1 to 0.45.

26. The gelling agent composition according to claim 23, wherein the (D) polyhydric alcohol is a polyhydric alcohol having 2 to 12 carbon atoms.

27. The gelling agent composition according to claim 23, wherein the (D) polyhydric alcohol is at least one type selected from the group consisting of butylene glycol, propylene glycol, propanediol, pentylene glycol, isopentyldiol, hexanediol, and dipropylene glycol.

28. The gelling agent composition according to claim 18, which is used for a solid cosmetic.

29. The gelling agent composition according to claim 28, wherein the solid cosmetic is a stick-shaped cosmetic.

30. The gelling agent composition according to claim 18, which melts at 100°C or lower.

31. A gelatinous composition comprising the gelling agent composition according to claim 18 and an oil agent.

32. The gelatinous composition according to claim 31, comprising 40 to 75% by weight of the gelling agent composition relative to the total weight of the gelatinous composition.

33. The gelatinous composition according to claim 31, having a rupture strength of 1500 g/cm² or more and 6500 g/cm² or less as measured at an insertion speed of 6 cm/min.

34. The gelatinous composition according to claim 31, which is a stick-shaped cosmetic.

35. A method for suppressing sweating in a solid cosmetic, comprising blending the gelling agent composition according to claim 1 in an oil agent.

36. A method for suppressing sweating in a solid cosmetic, comprising blending the gelling agent composition according to claim 2 or 18 in an oil agent.
